# EUROPEAN PATENT APPLICATION

(11) **EP 3 222 326 A1**
(43) Date of publication of application: **27.09.2017**
(21) Application number: 17161926.5
(22) Date of filing: 20.03.2017
(51) Int. Cl.: A61Q 11/00, A61K 8/19, A61K 8/24

(54) **TOOTHPASTE COMPOSITION**

(30) Priority: 23.03.2016 EP 16161806
(71) Applicant: Unilever PLC, London, Greater London EC4Y 0DY (GB); Unilever N.V., 3013 AL Rotterdam (NL)
(72) Inventor: BUGNI, Claudia, Bebington, Wirral, Merseyside CH63 3JW (GB); JOINER, Andrew, Bebington, Wirral, Merseyside CH63 3JW (GB); LIMER, Adam, John, Bebington, Wirral, Merseyside CH63 3JW (GB)
(74) Representative: Tansley, Sally Elizabeth

(57) **Abstract**

The present invention relates to a calcium source having a water solubility less than 0.1 moles per litre at room temperature in combination with a phosphate source for use in preventing calculus.

## Description

The present invention relates to materials that prevent the formation of calculus and their inclusion into toothpaste compositions.

Dental calculus is a deposit which forms on the surfaces of the teeth at the gingival margin. As the calculus develops, it becomes visibly white or yellowish in colour unless stained or discoloured by some extraneous agent. This is undesirable from an aesthetic standpoint.

WO9822080 (Procter and Gamble) discloses toothpastes that are said to have anti-tartar activity. The composition comprise polyphosphates.

The present invention discloses compositions with improved anti-calculus activity.

### Description of the Invention

The present invention relates to a calcium source having a solubility less than 0.1 moles per litre at room temperature in combination with a phosphate source for use in preventing calculus.

### Detailed Description of the Invention

The present invention relates to a toothpaste composition for use in preventing calculus, the composition comprising a water insoluble and/or slightly soluble calcium source and a phosphate source.

Compositions for use in the invention comprises a water insoluble and/or slightly soluble calcium source and a phosphate source.

Soluble and insoluble calcium source, as used herein, refers to the solubility of the calcium source in water. Soluble means a source that dissolves in water to give a solution with a concentration of at least 0.1 moles per litre at room temperature. Insoluble means a source that dissolves in water to give a solution with a concentration of less than 0.001 moles per litre at room temperature. Slightly soluble, therefore, is defined to mean a source that dissolves in water to give a solution with a concentration of greater than 0.001 moles per litre at room temperature and less than 0.1 moles per litre at room temperature. Substantially free of, as used herein, means less than 1.5%, and preferably, less than 1.0%, and most preferably, from 0.0 to 0.75% by weight, based on total weight of the oral care composition, including all ranges subsumed therein. Single-phase composition means a one phase composition having both calcium and phosphate sources therein and prior to dispensing or unpackaging and use. Anhydrous, as used herein, means substantially free of water. Generated calcium and phosphate comprising compound means a compound with calcium and phosphate like calcium phosphate and hydroxyapatite that is formed within the monophase product from distinct compounds comprising calcium and phosphate. Toothpaste compositions relates to pastes and gel compositions, preferably pastes.

The calcium source suitable for use in this invention is limited only to the extent that the same may be used in an oral cavity. In a preferred embodiment, the calcium source employed is insoluble or slightly soluble in water, but most preferably, insoluble in water.

Illustrative examples of the types of calcium source that may be used in this invention include, for example, calcium phosphate (i.e., added), calcium gluconate, calcium oxide, calcium lactate, calcium carbonate, calcium hydroxide, calcium sulfate, calcium carboxymethyl cellulose, calcium alginate, calcium salts of citric acid, calcium silicate, mixtures thereof or the like. In a preferred embodiment, the calcium source is calcium silicate. In a more preferred embodiment, the calcium silicate used is (CaSiO₃) whereby the same is made commercially available under the name Microcal ET by Ineos Silicas, Ltd.

In yet another preferred embodiment, the calcium source is insoluble calcium silicate, present as the composite material calcium oxide-silica (CaO-SiO₂) as described in commonly-owned application Publication No. 2008/015117.

When a calcium silicate composite material is employed, the ratio of calcium to silicon (Ca:Si) may be from 1:10 to 3:1. The Ca:Si ratio is preferably from 1:5 to 2:1, and more preferably, from 1:3 to 2:1, and most preferably, from about 1:2 to 2:1. The calcium silicate may comprise mono-calcium silicate, bi-calcium silicate, or tri-calcium silicate whereby ratios of calcium to silicon (Ca:Si) should be understood to be atom ratios.

The calcium source employed in this invention may be in a crystalline or amorphous state, and preferably, the same is in an amorphous state. In an often preferred embodiment, the calcium source is in a mesoporous state, i.e. the source is a material having pores with diameters from 1 nm to 50 microns. Mesoporous calcium silicate (MCS) is often preferred.

The MCS which may be used in this invention can be made by combining a calcium salt, a silica precursor like silicate and a structure-directing agent to yield a solid suitable for calcinating. A more detailed description of the process that may be conducted to make the MCS suitable for use in this invention is described in the aforementioned commonly-owned application, Publication No. WO 2008/015117.

The amount of calcium source in the composition of this invention is typically from 0.1 to 50%, and preferably, from 1 to 30%, and most preferably, from 5 to 20% by weight of the oral care composition based on total weight of the oral care composition and including all ranges subsumed therein.

The phosphate source that may be used in this invention is limited only to the extent that the same may be used in a composition suitable for use in an oral cavity. Illustrative examples of the types of phosphate source suitable for use in this invention include monosodium phosphate, sodium dihydrogen phosphate, disodium hydrogen phosphate, sodium pyrophosphate, tetrasodium pyrophosphate, sodium tripolyphosphate, sodium hexametaphosphate, potassium dihydrogenphosphate, trisodium phosphate, tripotassium phosphate, mixtures thereof or the like. The phosphate source is preferably one which is water soluble.

Typically, the phosphate source makes up from 0.5 to 15%, and preferably, from 2 to 12%, and most preferably, from 4 to 9% by weight of the oral care composition, based on total weight of the oral care composition and including all ranges subsumed therein. In a preferred embodiment, the phosphate source used is one which results in an oral care composition having a pH from 5.5 to 8, preferably from 6 to 7.5, and most preferably, about neutral. In a most preferred embodiment, the phosphate source used is trisodium phosphate and monosodium dihydrogen phosphate at a trisodium phosphate to monosodium dihydrogen phosphate weight ratio of 1:4 to 4:1, preferably 1:3 to 3:1, and most preferably, from 1:2 to 2:1, including all ratios subsumed therein.

The oral care compositions described herein may comprise ingredients which are common in the art, such as:
▪ antimicrobial agents, e.g. Triclosan, chlorhexidine, copper-, zinc- and stannous salts such as zinc citrate, zinc sulphate, zinc glycinate, sodium zinc citrate and stannous pyrophosphate, sanguinarine extract, metronidazole, quaternary ammonium compounds, such as cetylpyridinium chloride; bis-guanides, such as chlorhexidine digluconate, hexetidine, octenidine, alexidine; and halogenated bisphenolic compounds such as 2,2' methylenebis-(4-chloro-6-bromophenol);
▪ anti-inflammatory agents such as ibuprofen, flurbiprofen, aspirin, indomethacin, etc.;
▪ anti-caries agents such as sodium trimetaphosphate and casein;
▪ plaque buffers such as urea, calcium lactate, calcium glycerophosphate and strontium polyacrylates;
▪ vitamins such as Vitamins A, C and E;
▪ plant extracts;
▪ desensitising agents, e.g. potassium citrate, potassium chloride, potassium tartrate, potassium bicarbonate, potassium oxalate, and potassium nitrate;
▪ anti-calculus agents, e.g. alkali-metal pyrophosphates, hypophosphite-containing polymers, organic phosphonates and phosphocitrates, etc.;
▪ biomolecules, e.g. bacteriocins, antibodies, enzymes, etc.
▪ flavors, e.g., peppermint and spearmint oils;
▪ proteinaceous materials such as collagen;
▪ preservatives;
▪ opacifying agents;
▪ coloring agents like FD&C blue, yellow and/or red dyes/colorants;
▪ pH-adjusting agents;
▪ sweetening agents;
▪ surfactants, such as anionic, nonionic, cationic and zwitterionic or amphoteric surfactants (e.g., sodium lauryl sulfate, sodium dodecylbenzene sulfonate);
▪ particulate abrasive materials such as abrasive silicas, aluminas, calcium carbonates, zirconium silicate, polymethylmethacrylate, dicalciumphosphates, calcium pyrophosphates, hydroxyapatites, trimetaphosphates, insoluble hexametaphosphates as well as agglomerated particulate abrasive materials;
▪ fluoride sources like sodium fluoride, stannous fluoride, sodium monofluorophosphate, zinc ammonium fluoride, tin ammonium fluoride, calcium fluoride, cobalt ammonium fluoride or mixtures thereof;
▪ polymeric compounds which can enhance the delivery of active ingredients such as antimicrobial agents can also be included. Examples of such polymers are copolymers of polyvinylmethylether with maleic anhydride and other similar delivery enhancing polymers, e.g., those described in DE-A03,942,643;
▪ buffers and salts to buffer the pH and ionic strength of the oral care compositions; and
▪ other optional ingredients that may be included are, e.g., bleaching agents such as peroxy compound, e.g., potassium peroxydiphosphate, effervescing systems such as sodium bicarbonate/citric acid systems, colour change systems, and the like.

Such ingredients common in the art typically and collectively make-up less than 20% by weight of the oral care composition, and preferably, from 0.0 to 15% by weight, and most preferably, from about 0.01 to about 12% by weight of the oral care composition, including all ranges subsumed therein.

Thickener may also be used in this invention and is limited only to the extent that the same may be added to a composition suitable for use in an oral cavity. Illustrative examples of the types of thickeners that may be used in this invention include, sodium carboxymethyl cellulose, hydroxyl ethyl cellulose, methyl cellulose, ethyl cellulose, gum tragacanth, gum Arabic, gum karaya, sodium alginate, carrageenan, guar, xanthan gum, Irish moss, starch, modified starch, silica based thickeners including silica aerogels, magnesium aluminum silicate (i.e., Veegum) Carbomers (cross-linked acrylates) and mixtures thereof.

Typically, sodium carboxymethyl cellulose and/or Carbomers are preferred. When a Carbomer is employed, those having a molecular weight of at least 700,000 are desired, and preferably, those having a molecular weight of at least 1,200,000, and most preferably, those having a molecular weight of at least about 2,500,000 are desired. Mixtures of Carbomers may also be used herein.

In an especially preferred embodiment, the Carbomer is Carbopol^{®} 980. It has been described as a high molecular weight and cross-linked polyacrylic acid and identified via CAS number 9063-87-0. The same is available commercially from Lubrizol Advanced Materials, Inc.

Thickener typically makes up from 0.01 to about 10%, and preferably, from 0.1 to 8%, and most preferably, from 1.5 to 6% by weight of the oral care composition, based on total weight of the oral care composition and including all ranges subsumed therein.

Suitable carrier humectants are preferably used in the oral care composition of the present invention and they include, for example, glycerin, sorbitol, propylene glycol, dipropylene glycol, diglycerol, triacetin, mineral oil, polyethylene glycol (preferably, PEG-400), alkane diols like butane diol and hexanediol, ethanol, pentylene glycol, or a mixture thereof. The carrier humectants should, in any case, be substantially free of water, and preferably, anhydrous. The same, for example, can be used in solid form, whereby glycerin is the preferred carrier humectant.

The carrier humectant is used to take the balance of the compositions up to 100%, and the same may be present in the range of from 10 to 90% by weight of the oral care composition. Preferably, the carrier humectant makes up from 25 to 80%, and most preferably, from 45 to 70% by weight of the oral care composition, based on total weight of the oral care composition and including all ranges subsumed therein.

Preferably the composition is a single phase composition.

The compositions of this invention are prepared by conventional methods of making oral care formulations. Such methods include mixing the ingredients under moderate shear and atmospheric pressure. The compositions are used in the oral cavity, and preferably, are of the form that may be brushed onto teeth with a toothbrush.

The invention will now be demonstrated by the following non-limiting Examples:

### Examples

Oral care compositions (monophase) were prepared by mixing (in weight percent) the following ingredients under moderate shear until a homogeneous composition was obtained.

| **Ingredient** | **%** |
|---|---|
| Glycerin (99.5%) | To 100 |
| Calcium silicate | 15.0 |
| PEG 400 | 10,5 |
| Hydrated Silica | 6,0 |
| Trisodium Phosphate anhydrous | 3.8 |
| Monosodium Phosphate Anhydrous | 3.2 |
| Sodium Lauryl sulfate | 2.0 |
| PEG 3000S | 2.0 |
| Sodium Monofluorophosphate | 1.1 |
| Optamint 405213^{®} | 1.1 |
| TiO2 | 0.5 |
| Hydrated Silicon Dioxide | 0.5 |
| Sodium saccharin | 0.3 |
| Polyacrylic acid cross linked | 0.1 |

The composition of 1 was further mixed at a 1:1 ratio with a serum having the following formula:

| **Ingredients** | **%** |
|---|---|
| Glycerin (99.5%) | 53.8 |
| Calcium silicate | 6.4 |
| PEG 400 | 5.3 |
| Trisodium Phosphate anhydrous | 1.9 |
| Monosodium Phosphate Anhydrous | 1.6 |
| Sodium Lauryl sulfate (PAS) | 1.0 |
| PEG 3000S | 1.0 |
| Sodium Monofluorophosphate | 0.6 |
| Sodium Fluoride 98% | 0.2 |
| Water and minors | To 100 |

The product mix above was tested against a standard fluoride toothpaste comprising dicalcium Phosphate Dihydrate, Aqua, Glycerin, Sorbitol, PEG-12, Sodium Lauryl Sulfate, Aroma, Cellulose Gum, Sodium Monofluorophosphate, Tetrasodium Pyrophosphate, Sodium Saccharin, Calcium Glycerophosphate, Sodium Fluoride, Limonene

The products were tested using 218 panellists over a 6 week period. Products were assessed using a Volpe-manhold score summed across 6 specific sites.

Results showed that the subjects using the product of the invention had significantly less calculus than those using the conventional toothpaste.

## Claims

1. A calcium source having a solubility less than 0.1 moles per litre at room temperature in combination with a phosphate source for use in preventing calculus.

2. Use according to claim 1 in which the a water insoluble and/or slightly soluble calcium source and a phosphate source is formulated in a toothpaste composition.

3. Use according to claim 2 in which the composition is substantially free of water.

4. Use according to any preceding claim in which the phosphate source of the composition is water-soluble.

5. Use according to any preceding claim in which the calcium source of the composition comprises calcium gluconate, calcium oxide, calcium lactate, calcium carbonate, calcium hydroxide, calcium sulphate, calcium carboxymethyl cellulose, calcium alginate, calcium salts of citric acid, calcium silicate or a mixture thereof.

6. Use according to any of the preceding claims in which the calcium source is calcium silicate.

7. Use according to claim 6 in which the calcium silicate is one having a of Ca:Si ratio of 1:10 to 3:1.

8. Use according to any of the preceding claims wherein the phosphate source of the composition is monosodium phosphate, sodium dihydrogen phosphate, disodium hydrogen phosphate, sodium pyrophosphate, tetrasodium pyrophosphate, sodium tripolyphosphate, sodium hexametaphosphate, potassium dihydrogenphosphate, trisodium phosphate, tripotassium phosphate or a mixture thereof.

9. Use according to claim 8 wherein the phosphate source is trisodium phosphate, disodium hydrogen phosphate, monosodium dihydrogen phosphate or a mixture thereof.

10. Use according to any one of claims 2 to 9 in which the composition further comprises a thickener selected form the groups consisting of carboxymethyl cellulose, Carbomer or mixtures thereof.

11. Use according to any one of claims 2 to 10 in which the composition further comprises a carrier humectant selected from the group consisting of glycerin, sorbitol, propylene glycol, dipropylene glycol, diglycerol, triacetin, mineral oil, polyethylene glycol (preferably, PEG-400), alkane diols like butane diol and hexanediol, ethanol, pentylene glycol or mixtures thereof.

12. Use according to any one of claims 2 to 1 1 in which the composition has a pH from 5.5 to 8.
